# EUROPÄISCHE PATENTANMELDUNG

(11) **EP 1 298 218 A1**
(43) Veröffentlichungstag der Anmeldung: **02.04.2003**
(21) Anmeldenummer: 02021271.8
(22) Anmeldetag: 19.09.2002
(51) Int. Cl.: C12P 41/00

(54) **Verfahren zur Herstellung von enantiomerenreinen tertiären beta-Hydroxycarbonsäuren bzw. deren Estern**

(30) Priorität: 27.09.2001 DE 10147653
(71) Anmelder: Consortium für elektrochemische Industrie GmbH, 81379 München (DE)
(72) Erfinder: Popp, Alfred, Dr., 82008 Unterhaching (DE); Petersen, Hermann, Dr., 84489 Burghausen (DE); Stohrer, Jürgen, Dr., 82049 Pullach (DE); Rockinger-Mechlem, Jodoca, 82205 Gilching (DE); Gilch, Andrea, 85419 Mauern (DE)
(74) Vertreter: Potten, Holger

(57) **Zusammenfassung**

Verfahren zur Herstellung einer enantiomerenreinen tertiären β-Hydroxycarbonsäure bzw. ihres Esters dadurch gekennzeichnet, daß ein Enantiomerengemisch von Verbindungen mit einem Enzym, das zur hydrolytischen Spaltung einer Esterbindung befähigt ist, in einem wässrigen Medium in Kontakt gebracht wird, so daß ein Enantiomer des Enantiomerengemisches hydrolysiert wird.

## Beschreibung

Die Erfindung betrifft ein Verfahren zur Herstellung von enantiomerenreinen tertiären β-Hydroxycarbonsäuren bzw. deren Estern.

Enantiomerenreine Derivate dienen als Ausgangsmaterialien bzw. Zwischenprodukte bei der Synthese von Agrochemikalien und Pharmazeutika. Viele dieser Verbindungen werden zur Zeit als Racemat oder Diastereomerengemisch hergestellt und vermarktet. In vielen Fällen wird der gewünschte physiologische Effekt aber nur von einem Enantiomer / Diastereomer bewirkt. Das andere Isomer ist im günstigsten Fall inaktiv, es kann aber auch dem gewünschten Effekt entgegenwirken oder sogar toxisch sein. Verfahren zur Trennung von Racematen werden deshalb immer wichtiger für die Darstellung hochenantiomerenreiner Verbindungen. Es ist bekannt, dass die Racemattrennung chiraler Verbindungen mit Hilfe von Enzymen durchgeführt werden kann. In einer Vielzahl von Publikationen werden enzymatisch kinetische Racematspaltungen von Estern mit Lipasen und Esterasen beschrieben. Die Racematspaltung von sekundären Alkoholen wird im allgemeinen durch Acylierung der Hydroxylgruppe am stereogenen Zentrum oder umgekehrt durch Hydrolyse des korrespondierenden Esters durchgeführt. Ist neben der Hydroxyfunktion noch eine zweite funktionelle Gruppe vorhanden, kann die selektive Trennung der Enantiomere auch durch Reaktion an dieser erreicht werden. Handelt es sich bei dieser zweiten Funktionalität um ein Carbonsäurederivat, so wurde bisher die Herstellung zweier großer Verbindungsklassen beschrieben: die enantiomerenreinen sekundären α-oder β-Hydroxycarbonsäuren und die tertiären α-Hydroxycarbonsäuren.

EP 459455 (Erfinder: Miyazawa, K. et al.) beschreibt eine Methode zur Racematspaltung von sekundären α-Hydroxyestern durch Umesterung in Gegenwart einer aus Pseudomonasspezies hergestellten Lipase unter im wesentlichen wasserfreien Bedingungen.

EP 391345 (Erfinder: Murakami, N. et al.) beschreibt eine Methode zur optischen Trennung von sekundären β-Hydroxyestern durch stereoselektive Hydrolyse der Esterfunktion in Gegenwart von Mikroorganismen. Die Verfahren von H. Yano (US 5643793) zur Herstellung von enantiomerenreiner 3-Hydroxyhexansäure unter Verwendung von *Porcine Pancreas Lipase (PPL)*, von A. Tixidre (EP 736606) zur Herstellung von Ethyl 4,4,4-Trifluor-3-(*R*)-hydroxybutanoat durch Hydrolyse der Esterfunktion mit einem Enzym aus *Candida antarctica* und der Trennung der optischen Isomere von sekundären Arylalkyl-β-Hydroxycarbonsäureestern unter Verwendung von *Pseudomonas Fluorescens Lipase (PFL)* von N. W. Boaz (EP 494203) beruhen auf dem selben Prinzip, wobei entweder das gewünschte oder das unerwünschte Stereoisomer hydrolysiert wird und dadurch eine Trennung und anschließende Isolierung der beiden optischen Antipoden möglich ist.

EP 512848 (Erfinder: Yee, Ch. et al.) und EP 786012 (Erfinder: Sariaslani, F. et al.) beschreiben eine Methode zur optischen Trennung von tertiären α-Hydroxycarbonsäureestern. Dabei beschränken sich Yee et al. auf die Verwendung eines Enzyms aus *Candida lipolytica*, wohingegen Sariaslani et al. die gleiche Reaktion mit einem breiten Spektrum verschiedener hydrolytisch wirksamer Enzyme durchführen.

Demgegenüber sind zur Herstellung von enantiomerenreinen tertiären β-Hydroxycarbonsäuren bzw. deren Estern nur wenige Arbeiten bekannt. Diese beschränken sich auf zwei Spezialfälle. Zum einen wird die Desymmetrisierung von meso-Diestern wie z. B. β-Hydroxy-β-methyl-glutarsäuredimethylester (Huang, F. et al. *J.* Am. *Chem. Soc*. 1975, 97(14), S. 4144-4145; Toone, E. et al. *J. Am. Chem. Soc*. 1990, 112(12), S. 4946-4952) und zum anderen die enantioselektive Hydrolyse von 3-Hydroxy-3-methylalkansäureestern mit Schweineleberesterase (PLE) beschrieben (Wilson, W. K. et al. *J. Org. Chem*. 1983, 48(22), S. 3960-3966). Nachteile dieser letzteren Methode sind die äußerst geringe Selektivität (E = 2.4 - 9; Definition von E s. Chen, C. et al. *J. Am. Chem. Soc*. 1982, 104, S. 7294-7299), die niedrigen erzielten Enantiomerenreinheiten der Produkte und die geringen chemischen Ausbeuten.

Eine optimale Racematspaltung sollte vorteilhafterweise folgende Bedingungen erfüllen:
1. hohe Enantiomerenreinheit der optischen Antipoden
2. hohe chemische Ausbeute
3. hohe Selektivität des Enzyms
4. gute Raum-Zeit-Ausbeuten
5. kostengünstige Synthese

Aufgabe der vorliegenden Erfindung ist es, ein Verfahren zur Herstellung einer enantiomerenreinen tertiären β-Hydroxycarbonsäure (Formel Ia oder Ib) bzw. ihres Esters (Formel IIa oder IIb) zur Verfügung zu stellen, welches kostengünstig ist und die genannten Nachteile vermeidet, 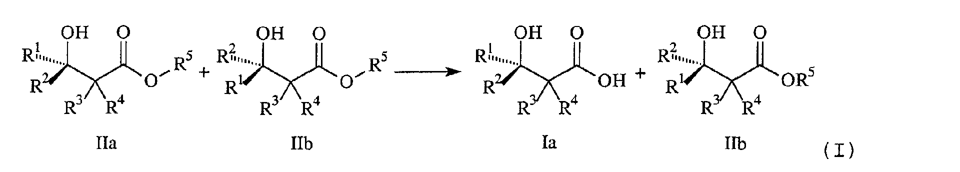 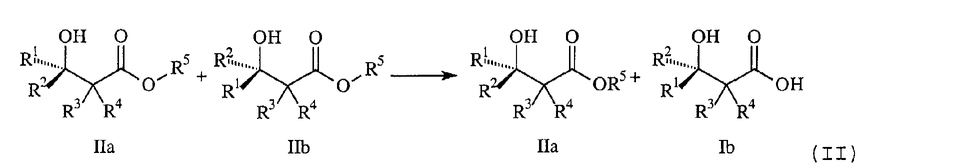 wobei die Substituenten folgende Bedeutung besitzen:
R¹ ungleich R², wobei R¹ und R² unabhängig voneinander substituiertes oder unsubstituiertes C₆-C₁₈-Aryl, C₃-C₁₈-Heteroaryl, C₂-C₁₈-Alkyl, C₂-C₁₈-Alkenyl, C₂-C₁₈-Alkinyl, C₆-C₁₈-Aryl-C₁-C₁₈-Alkyl, C₃-C₁₈-Heteroaryl-C₁-C₁₈-Alkyl, C₆-C₁₈-Aryl-C₂-C₁₈-Alkenyl, C₃-C₁₈-Heteroaryl-C₂-C₁₈-Alkenyl, C₁-C₁₈-Alkoxy-C₁-C₁₈-Alkyl, C₁-C₁₈-Alkoxy-C₂-C₁₈-Alkenyl, C₆-C₁₈-Aryloxy-C₁-C₁₈-Alkyl, C₆-C₁₈-Aryloxy-C₂-C₁₈-Alkenyl, C₃-C₈-Cycloalkyl, C₃-C₈-Cycloalkyl-C₁-C₁₈-Alkyl, C₃-C₈-Cycloalkyl-C₂-C₁₈-Alkenyl bedeuten oder R¹ und R² zusammen mit dem Kohlenstoff an den sie gebunden sind, ein substituiertes, unsubstituiertes oder Heteroatom-enthaltendes Cycloalkyliden bilden,
R³ und R⁴ gleich oder verschieden sind, wobei R³ und R⁴ unabhängig voneinander substituiertes oder unsubstituiertes C₆-C₁₈-Aryl, C₃-C₁₈-Heteroaryl, C₁-C₁₈-Alkyl, C₂-C₁₈-Alkenyl, C₂-C₁₈-Alkinyl, C₆-C₁₈-Aryl-C₁-C₁₈-Alkyl, C₃-C₁₈-Heteroaryl-C₁-C₁₈-Alkyl, , C₆-C₁₈-Aryl-C₂-C₁₈-Alkenyl, C₃-C₁₈-Heteroaryl-C₂-C₁₈-Alkenyl, C₁-C₁₈-Alkoxy-C₁-C₁₈-Alkyl, C₁-C₁₈-Alkoxy-C₂-C₁₈-Alkenyl, C₆-C₁₈-Aryloxy-C₁-C₁₈-Alkyl, C₆-C₁₈-Aryloxy-C₂-C₁₈-Alkenyl, C₃-C₈-Cycloalkyl, C₃-C₈-Cycloalkyl-C₁-C₁₈-Alkyl, C₃-C₈-Cycloalkyl-C₂-C₁₈-Alkenyl bedeuten oder R³ und R⁴ zusammen mit dem Kohlenstoff an den sie gebunden sind, ein substituiertes, unsubstituiertes oder Heteroatom-enthaltendes Cycloalkyliden bilden,
R⁵ substituiertes oder unsubstituiertes C₁-C₁₈-Alkyl oder C₁-C₁₈-Alkenyl.

Soweit es sich bei den Resten um substituierte Reste handelt, sind diese vorzugsweise durch Alkyl-, Alkenyl-, Alkinyl-, Aryl-, Heteroaryl-, Hydroxy-, Alkoxy-, Carboxylat-, Alkoxycarbonyl-, Amino-, Nitro- oder Halogenreste substituiert.

Soweit die vorstehend genannten Reste ein Heteroatom enthalten, handelt es sich dabei vorzugsweise um O, N oder S.

Die Aufgabe wird gelöst durch ein Verfahren, welches dadurch gekennzeichnet ist, daß ein Enantiomerengemisch von Verbindungen der Formel II mit einem Enzym, das zur hydrolytischen Spaltung einer Esterbindung befähigt ist, in einem wässrigen Medium in Kontakt gebracht wird, so daß ein Enantiomer des Enantiomerengemisches hydrolysiert wird.

Bei dem Enantiomerengemisch von Verbindungen der Formel II handelt es sich bevorzugt um ein Enantiomerengemisch der allgemeinen Formel (III), 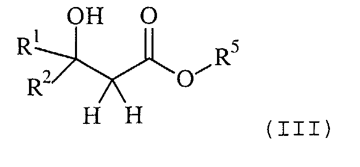 wobei R¹, R² und R⁵ die bereits genannte Bedeutung haben

Besonders bevorzugt handelt es sich um ein Enantiomerengemisch der allgemeinen Formel (IV), 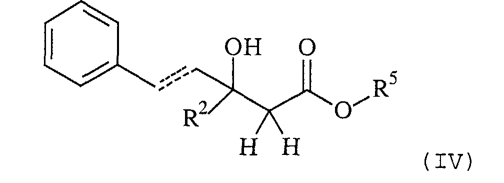 wobei R² und R⁵ die bereits genannte Bedeutung besitzen und die gestrichelte Bindung entweder eine Einfach- oder Doppelbindung repräsentiert.

Für das erfindungsgemäße Verfahren sind prinzipiell alle Enzyme, die zur Spaltung einer Esterbindung befähigt sind, geeignet. Bevorzugt handelt es sich um eine Lipase oder Esterase der Klasse 3.1 gemäß Internationaler Enzym-Nomenklature, Committee of the International Union of Biochemistry and Molecular Biology. Wegen ihrer einfacheren Zugänglichkeit besonders bevorzugt handelt es sich um Lipasen oder Esterasen mikrobiellen Ursprungs, Schweinepankreaslipase oder Pferde-/ Schweineleberesterase. Als Enzyme mikrobiellen Ursprungs seien beispielsweise Enzyme aus Pilzen, Hefen oder Bakterien wie beispielsweise von Alcaligenes sp., Aspergillus niger, Aspergillus oryzae, Bacillus sp., Bacillus stearothermophilus, Bacillus thermoglucosidasius, Candida antarctica, Candida lipolytica, Candida rugosa, Chromobacterium viscosum, Geotrichum scandium, Mucor miehei, Penicillium camembertii, Penicillium roquefortii, Pseudomonas cepacia, Pseudomonas fluorescens, Pseudomonas sp., Rhizomucor javanicus, Rhizopus arrhizus, Rhizopus niveus, Saccharomyces cerevisiae, Thermoanaerobium brockii, Thermomyces lanuginosa genannt.

Besonders bevorzugt werden Lipasen und Esterasen aus Candida-Arten wie zum Beispiel Candida antarctica B und Schweineleberesterase.

Als Enzym ganz besonders bevorzugt sind Novozym® 435, 525 (Firma Novo, Dänemark) und Chirazyme® L2, E1, E2 (Firma Böhringer Mannheim, Deutschland).

Die Enzyme werden in der Reaktion direkt oder als Immobilisate an unterschiedlichsten Trägern eingesetzt. Die Immobilisate können hergestellt werden durch Lösen des Enzyms in einem Puffer bei geeignetem pH und anschließender passiver Adsorption an den Träger wie z. B. Diatomeenerde (Celite®), Aktivkohle, Aluminiumoxid, Kieselgel, Kieselguhr, monodispers lösliche Organosiloxanpartikel oder Harze (z. B. Amberlite®, Dowex®). Alternativ können die Enzyme auch kovalent an den Träger gebunden werden (z. B. Polystyrol oder Epoxy-Harze wie Eupergit®). Die geträgerten Enzyme können durch Lyophilisieren getrocknet werden.

Die zuzusetzende Menge Enzym hängt von der Art des Edukts, Produkts und der Aktivität der Enzympräparation ab. Die für die Reaktion optimale Enzymmenge kann leicht durch einfache Vorversuche ermittelt werden. Je nach Enzym liegt das Enzym-Substratverhältnis berechnet als Molverhältnis zwischen Enzym und Substrat in der Regel zwischen 1 : 1000 bis 1 : 50000000, bevorzugt bei 1 : 10000 bis 1 : 5000000. Die Enantioselektivität E der Enzyme liegt dabei in der Regel zwischen 5 und > 100. Bevorzugt ist die Enantioselektivität größer als 10.

Als wässriges Medium für die Hydrolysereaktion dient vorzugsweise Wasser. Bevorzugt wird ein vorgegebener pH-Wert der wässrigen Phase durch Zugabe eines Puffers eingestellt. Besonders bevorzugt verwendet wird ein Na₂HPO₄/ NaH₂PO₄-Puffer mit einem pH von 7.0.

Um während der Reaktion den pH konstant zu halten, kann auch eine wässrige Lauge, bevorzugt die Lösung eines Alkalihydroxyds in Wasser, besonders bevorzugt die wässrige Lösung von NaOH oder KOH, zudosiert werden.

Die Enzymreaktion kann ohne Zugabe zusätzlicher organischer Lösungsmittel oder Lösungsmittelgemische als Suspension oder Emulsion in Wasser, respektive Puffer, als Reaktionsmedium durchgeführt werden, wobei konventionelle Emulgiermittel die Emulsionsbildung verbessern können.

Vorteilhafterweise werden der Reaktion weitere Lösungsmittel oder Lösungsmittelgemische zugesetzt. Prinzipiell eigenen sich hierfür alle aprotischen oder protogenen Lösungsmittel. Geeignet sind alle Lösungsmittel, die in der Reaktion inert sind, das heißt, sie dürfen an der Enzymreaktion nicht beteiligt sein. Ungeeignet sind beispielsweise Lösungsmittel, in deren Gegenwart Nebenreaktionen auftreten können, da sie selber Enzymsubstrate sind (z. B. Ester primärer und sekundärer Alkohole). Als geeignete Lösungsmittel seien hier beispielsweise reine aliphatische oder aromatische Kohlenwasserstoffe wie Hexan, Cyclohexan, Petrolether oder Toluol, halogenierte Kohlenwasserstoffe wie Methylenchlorid oder Chloroform, Ether wie Methyl*tert*.-Butylether (MTBE), Tetrahydrofuran, Diethylether, Diisopropylether oder Dioxan, tertiäre Alkohole wie tert.-Butanol, tert. Pentylalkohol, Ester von tertiären Alkoholen wie tert.-Butylacetat oder Acetonitril genannt. Als bevorzugte Lösungsmittel seien Methyl-tert.-Butylether (MTBE) oder Diisopropylether genannt.

Die Reaktion wird vorteilhafterweise bei einer Temperatur zwischen 0 °C und 75 °C durchgeführt, bevorzugt zwischen 10 °C und 60 °C, besonders bevorzugt zwischen 20 °C und 50 °C.

Die Reaktionszeiten betragen je nach Substrat, Ester und Enzymart und -menge zwischen 10 Minuten und 7 Tage. Bevorzugt liegen die Reaktionszeiten zwischen 1 und 48 Stunden.

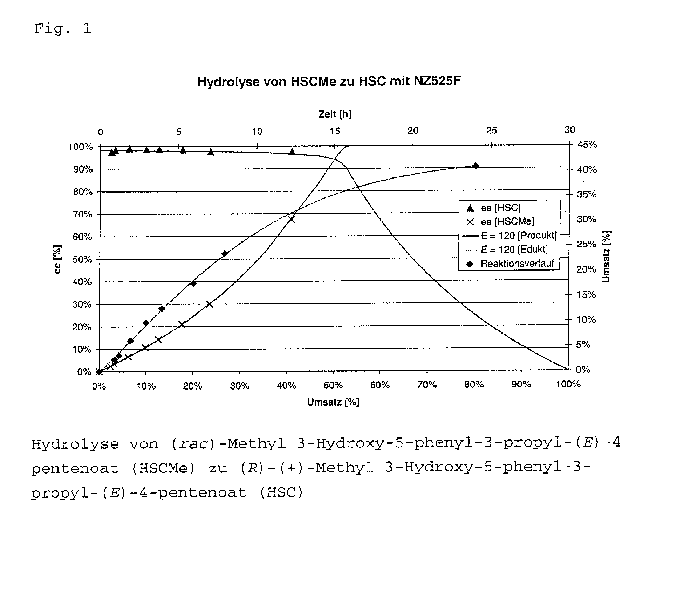

Der Reaktionsverlauf läßt sich leicht mit üblichen Methoden beispielsweise dem Laugenverbrauch bei der pH-Stat-Titration oder HPLC verfolgen. Die Reaktion kann je nach gewünschtem Ergebnis (hohe Umsetzung, hoher Enantiomerenüberschuß des Substrats oder des Produkts, s. Fig. 1) beendet werden. Im Idealfall ist die Reaktion bei einem Umsatz von 50 % bei einer hohen Enantiomerenreinheit sowohl im Substrat als auch im Produkt beendet (Fig. 1). Gestoppt wird die Reaktion durch Separation des nicht umgesetzten Enantiomers des Enantiomerengemisches bzw. des Produkts der enzymatischen Umsetzung, z. B. durch Extraktion der wässrigen Phase oder Destillation.

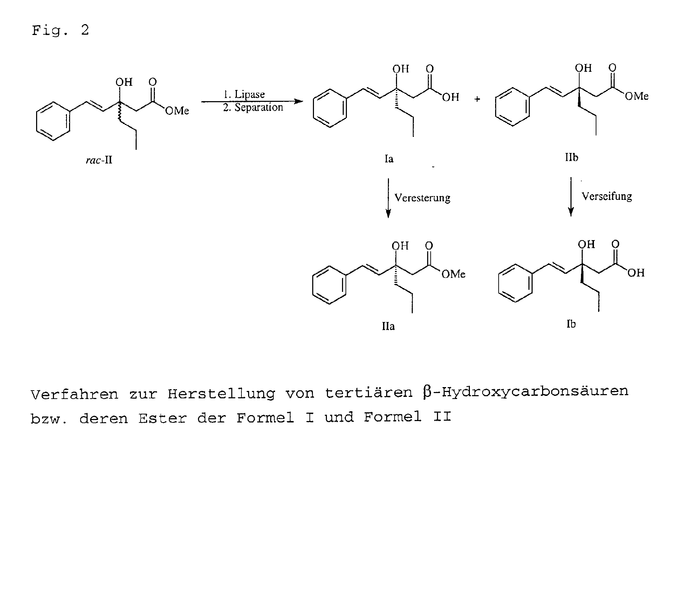

Je nach Enzym wird das (*R*)- bzw. (*S*)-Stereoisomer (siehe Formel II: IIa oder IIb) des Esters hydrolysiert und die korrespondierende freie Säure (siehe Formel I: Ia oder Ib) selektiv gebildet. Das jeweils andere Enantiomer wird nicht umgesetzt und bleibt unverändert auf der Esterstufe zurück. Fig. 2 zeigt beispielhaft die Synthese für ein Enantiomer der Säure Ia und des korrespondierenden Esters IIb mit entgegengesetzter Chiralität und die weiteren Syntheseverfahren zur Umwandlung der erhaltenen Säure bzw. Ester in die gewünschte Form, also Ester IIa respektive Säure Ib.

Handelt es sich bei der in der Reaktion (Fig. 2) entstehenden Säure (Ia) um das gewünschte Enantiomer, so wird zunächst der verbleibende Ester (IIb) abgetrennt (z. B. durch Extraktion bei alkalischen pH) und anschließend die gewünschte Säure isoliert (z. B. durch Extraktion bei saurem pH).

Handelt es sich bei der in der Reaktion (Fig. 2) entstehenden Säure (Ia) um das unerwünschte Enantiomer, so kann der verbleibende Ester (IIb, das erwünschte Enantiomer) direkt abgetrennt werden (z. B. durch Extraktion bei alkalischen pH).

Die Säure- (Ia)/ bzw. Esterfunktion (IIb) der Reinenantiomere kann im Anschluß an die Enantiomerentrennung durch einfache chemische Synthesen (Verseifung, Veresterung) in die Gewünschte Form (IIa bzw. Ib) überführt werden.

Die folgenden Beispiele dienen der Veranschaulichung der vorliegenden Erfindung.

### Beispiel 1

Ein 500 mL Dreihalskolben mit KPG-Rührer, pH-Elektrode und Zulauf für eine Bürette wird mit 380 mL Wasser befüllt, 19.2 g (77.3 mmol) (*rac*)-Methyl 3-Hydroxy-5-phenyl-3-propyl-(*E*)-4-pentenoat werden darin unter starkem Rühren suspendiert und das Ganze auf 40 °C erwärmt. Durch Zugabe von 4.0 mL Novozym® 525F wird die Reaktion gestartet. Der pH wird durch kontinuierliche Zugabe von 2.0 N NaOH konstant gehalten. Das Ende der Reaktion wird durch HPLC-Analytik festgestellt.
Der pH-Wert der enzymhaltigen Lösung wird dann mit 1N NaOH auf 8 eingestellt und dann die Lösung mit 3 mal 200 mL MTBE extrahiert. Die vereinigten org. Phasen werden über Na₂SO₄ getrocknet, filtriert und unter vermindertem Druck eingeengt. Der Rückstand enthält (*R*)-(+)-Methyl 3-Hydroxy-5-phenyl-3-propyl-(*E*)-4-pentenoat (zähes Öl; Ausbeute: 9.3 g (37.4 mmol, 48 %) ; [α]_{D}²⁰ = +8.8 (c = 10, CHCl₃) ; ee = 72%).
Die verbleibende alkalische wäßrige Lösung wird mit 1N H₂SO₄ auf pH = 2 eingestellt und 3 mal mit 200 mL MTBE extrahiert. Die vereinigten organische Phasen werden über Na₂SO₄ getrocknet, filtriert und unter vermindertem Druck eingeengt. Der Rückstand enthält (*S*)-(-)- 3-Hydroxy-5-phenyl-3-propyl-(*E*)-4-pentensäure (farbloses Pulver; Ausbeute: 5.13 g (21.9 mmol, 28 %); Smp.: 86-87°C, [α]_{D}²⁰ = -7.7 (c = 10, CHCl₃) ; ee = 96%).

### Beispiele 2 bis 5

Analog Beispiel 1 wurden Racematspaltungen mit den in Tab. 1 wiedergegebenen Komponenten durchgeführt. Die Selektivität gibt die Effizienz der Reaktion an.

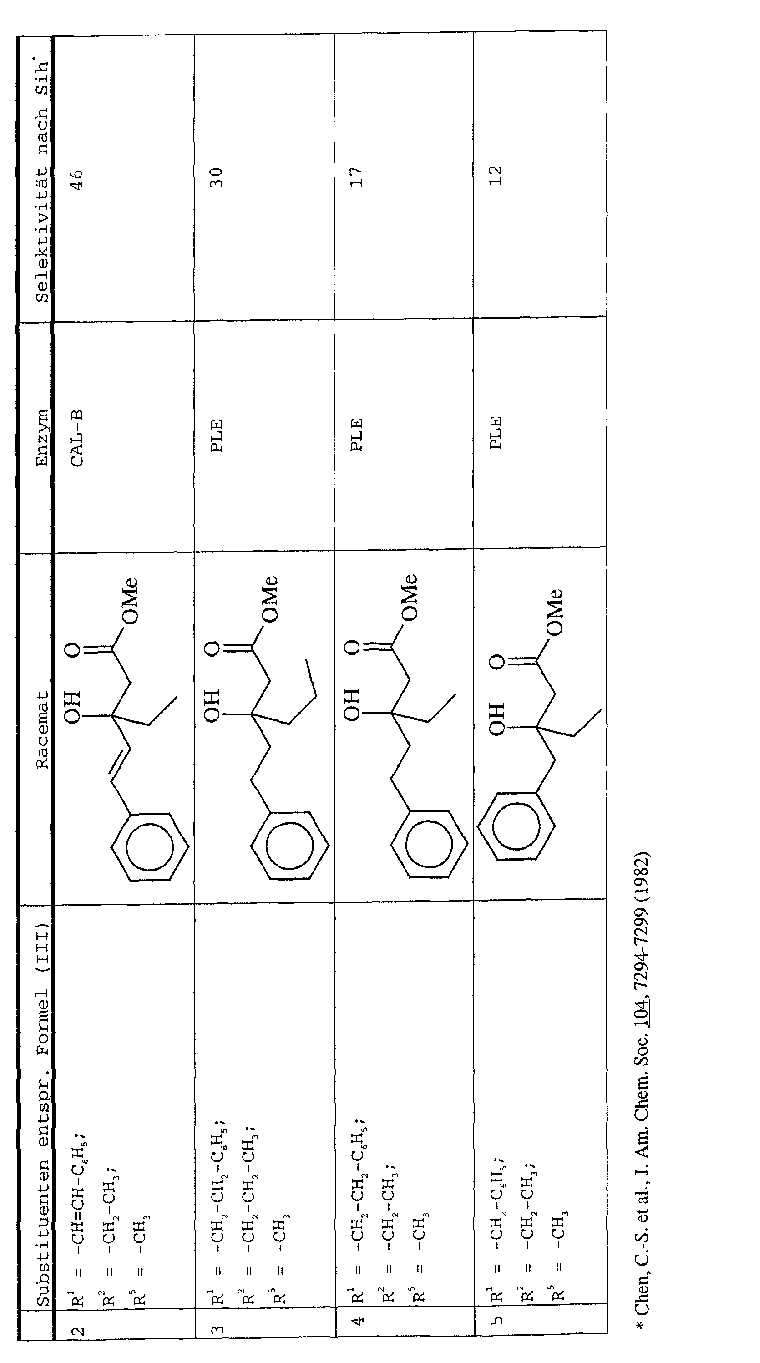

## Patentansprüche

1. Verfahren zur Herstellung einer enantiomerenreinen tertiären β-Hydroxycarbonsäure (Formel Ia oder Ib) bzw. ihres Esters (Formel IIa oder IIb) 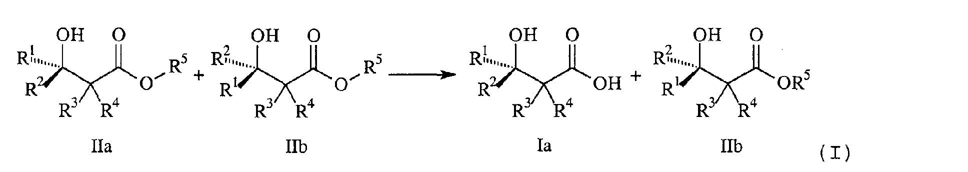 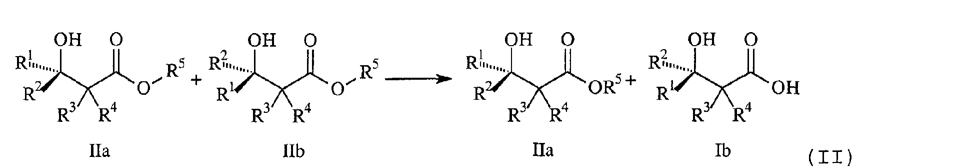 wobei die Substituenten folgende Bedeutung besitzen:
R¹ ungleich R², wobei R¹ und R² unabhängig voneinander substituiertes oder unsubstituiertes C₆-C₁₈-Aryl, C₃-C₁₈-Heteroaryl, C₂-C₁₈-Alkyl, C₂-C₁₈-Alkenyl, C₂-C₁₈-Alkinyl, C₆-C₁₈-Aryl-C₁-C₁₈-Alkyl, C₃-C₁₈-Heteroaryl-C₁-C₁₈-Alkyl, C₆-C₁₈-Aryl-C₂-C₁₈-Alkenyl, C₃-C₁₈-Heteroaryl-C₂-C₁₈-Alkenyl, C₁-C₁₈-Alkoxy-C₁-C₁₈-Alkyl, C₁-C₁₈-Alkoxy-C₂-C₁₈-Alkenyl, C₆-C₁₈-Aryloxy-C₁-C₁₈-Alkyl, C₆-C₁₈-Aryloxy-C₂-C₁₈-Alkenyl, C₃-C₈-Cycloalkyl, C₃-C₈-Cycloalkyl-C₁-C₁₈-Alkyl, C₃-C₈-Cycloalkyl-C₂-C₁₈-Alkenyl bedeuten oder R¹ und R² zusammen mit dem Kohlenstoff an den sie gebunden sind, ein substituiertes, unsubstituiertes oder Heteroatom-enthaltendes C₃-C₈-Cycloalkyliden bilden,
R³ und R¹ gleich oder verschieden sind wobei R³ und R⁴ unabhängig voneinander substituiertes oder unsubstituiertes C₆-C₁₈-Aryl, C₃-C₁₈-Heteroaryl, C₁-C₁₈-Alkyl, C₂-C₁₈-Alkenyl, C₂-C₁₈-Alkinyl, C₆-C₁₈-Aryl-C₁-C₁₈-Alkyl, C₃-C₁₈-Heteroaryl-C₁-C₁₈-Alkyl, C₆-C₁₈-Aryl-C₂-C₁₈-Alkenyl, C₃-C₁₈-Heteroaryl-C₂-C₁₈-Alkenyl, C₁-C₁₈-Alkoxy-C₁-C₁₈-Alkyl, C₁-C₁₈-Alkoxy-C₂-C₁₈-Alkenyl, C₆-C₁₈-Aryloxy-C₁-C₁₈-Alkyl, C₆-C₁₈-Aryloxy-C₂-C₁₈-Alkenyl, C₃-C₈-Cycloalkyl, C₃-C₈-Cycloalkyl-C₁-C₁₈-Alkyl, C₃-C₈-Cycloalkyl-C₂-C₁₈-Alkenyl bedeuten oder R³ und R⁴ zusammen mit dem Kohlenstoff an den sie gebunden sind, ein substituiertes, unsubstituiertes oder Heteroatom-enthaltendes C₃-C₈-Cycloalkyliden bilden,
R⁵ substituiertes oder unsubstituiertes C₁-C₁₈-Alkyl oder C₁-C₁₈-Alkenyl
**dadurch gekennzeichnet, daß** ein Enantiomerengemisch von Verbindungen der Formel II mit einem Enzym, das zur hydrolytischen Spaltung einer Esterbindung befähigt ist, in einem wässrigen Medium in Kontakt gebracht wird, so daß ein Enantiomer des Enantiomerengemisches hydrolysiert wird.

2. Verfahren gemäß Anspruch 1, **dadurch gekennzeichnet, daß** das zur hydrolytischen Spaltung einer Esterbindung befähigte Enzym eine Lipase oder Esterase ist.

3. Verfahren gemäß Anspruch 2, **dadurch gekennzeichnet, daß** das zur hydrolytischen Spaltung befähigte Enzym eine Candida antarctica Lipase Typ B oder eine Esterase aus Schweineleber ist.

4. Verfahren gemäß einem der Ansprüche 1 bis 3, **dadurch gekennzeichnet, daß** das Enzym in einem Enzym-Substratverhältnis berechnet als Molverhältnis zwischen Enzym und Substrat von 1 : 1000 bis 1 : 50000000 zugesetzt wird.

5. Verfahren gemäß einem der Ansprüche 1 bis 3, **dadurch gekennzeichnet, daß** das wässrige Medium ein wässriger Puffer ist.

6. Verfahren gemäß Anspruch 5, **dadurch gekennzeichnet, daß** man dem wässrigen Puffer ein inertes Lösungsmittel zusetzt.

7. Verfahren gemäß Anspruch 6, **dadurch gekennzeichnet, daß** das inerte Lösungsmittel Methyl-tert.-Butylether oder Diisopropylether ist.

8. Verfahren gemäß einem der Ansprüche 1 bis 7, **dadurch gekennzeichnet, daß** es bei Temperaturen von 20 bis 50 °C durchgeführt wird.

9. Verfahren gemäß einem der Ansprüche 1 bis 8, **dadurch gekennzeichnet, daß** die Reaktion durch Separation des nicht umgesetzten Enantiomers des Enantiomerengemisches oder des Produkts der enzymatischen Umsetzung gestoppt wird.

10. Verfahren gemäß Anspruch 9, **dadurch gekennzeichnet, daß** die Reaktion durch Extraktion der wässrigen Phase oder Destillation gestoppt wird.

11. Verfahren nach Anspruch 9, **dadurch gekennzeichnet, daß** die Säure- (Ia) bzw. Esterfunktion (IIb) oder die Säure- (IIa) bzw. Esterfunktion (Ib) der Enantiomere im Anschluß an die Separation durch einfache chemische Synthesen in die gewünschte Form (IIa bzw. Ib oder IIb bzw. Ia) überführt wird.

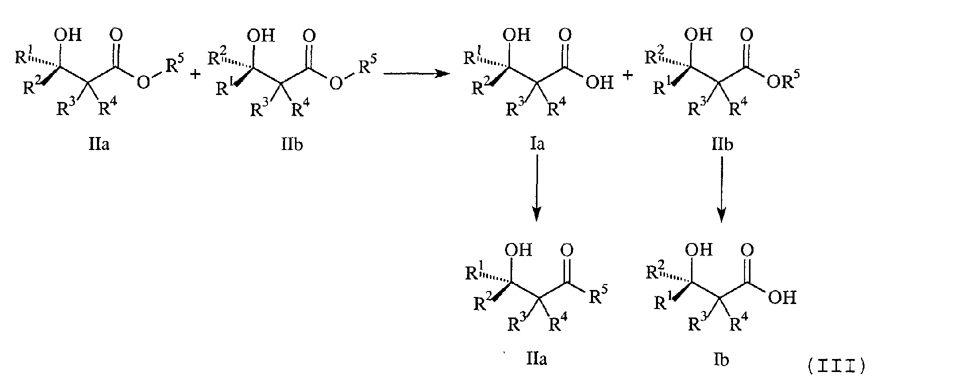

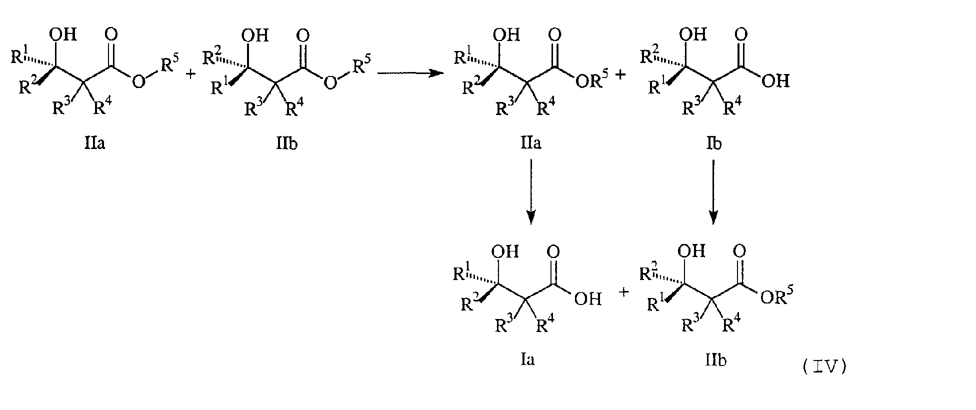

12. Verfahren gemäß Anspruch 11, **dadurch gekennzeichnet, daß** die einfache chemische Synthese eine Verseifung oder Veresterung darstellt.

13. Verfahren gemäß einem der Ansprüche 1 bis 13, **dadurch gekennzeichnet, daß** das Substrat für die enzymatische Hydrolyse Methyl 3-Hydroxy-5-phenyl-3-propyl-(*E*)-4-pentenoat ist.
